# EUROPEAN PATENT APPLICATION

(11) **EP 4 202 951 A1**
(43) Date of publication of application: **28.06.2023**
(21) Application number: 21290094.8
(22) Date of filing: 21.12.2021
(51) Int. Cl.: G16H 40/60, A61B 90/00

(54) **IMAGE-GUIDED THERAPY SYSTEM**

(71) Applicant: Koninklijke Philips N.V., 5656 AG Eindhoven (NL)
(72) Inventor: Haase, Hans Christian, 5656 AE Eindhoven (NL); Florent, Raoul, 5656 AE Eindhoven (NL)
(74) Representative: Philips Intellectual Property & Standards

(57) **Abstract**

The invention is directed towards an improved image-guided therapy system.

## Description

### FIELD OF THE INVENTION

The invention is directed to an image-guided therapy system, a computer-implemented method for image processing, a computer program and an imaging device.

### BACKGROUND OF THE INVENTION

In general, the medical imaging systems are used for Percutaneous Intervention in catheter laboratories to treat patients, for example Percutaneous Coronary Intervention (PCI) in catheter laboratories to treat cardiac stenosis patients. Typically, a catheter is inserted into the vascular system at an access site, it is advanced along large vessels to the vascular structure that requires treatment. Contrast agent is injected via the catheter and catheter laboratory x-ray equipment records an angiographic sequence that shows the vessels when filled with contrast agent. In general, diagnosis and intervention planning are based on such diagnostic angiograms. During intervention, a flexible, partially or fully radio-opaque guidewire is advanced to the affected vascular structures (e.g. stenosis in coronaries, neurovascular aneurisms, or arterio-venous malformations). Fluoroscopic low-dose x-ray surveillance visualizes the guidewire and allows for the hand-eye coordination of the interventionalist while advancing the guidewire. When positioned, the guidewire serves as a rail to deliver interventional devices (e.g. balloons for dilation and stent delivery, detachable coils for aneurysm clotting).

In the field of radiology, automatically positioning of a diagnostic imaging system is a complex subject, since not only the position of the patient has to be assessed but in addition the image quality of the medical image data has to be sufficient. In addition, medical imaging during an intervention is usually carried out manually since it is a very complex procedure. Usually, a plurality of guidelines for positioning the medical imaging device are provided for assisting medical personnel.

### SUMMARY OF THE INVENTION

With embodiments of the invention, an improved image-guide therapy system is provided.

The invention is defined by the independent claims. Further embodiments and advantages of the present invention are incorporated in the dependent claims and the description.

Technical terms are used by common sense. If a specific meaning is conveyed to certain terms, definition of terms will be given in the following in the context which the terms are used.

According to a first aspect of the invention, there is provided a method of training a machine learning (ML) algorithm for assisting image-guided therapy procedures (also known as interventions), for example image-guided therapy procedures using X-ray imaging like fluoroscopy and/or using cross-sectional digital imaging modalities such as Magnetic Resonance Imaging (MRI) and/or Computed Tomography (CT), optionally supported by auxiliary equipment such as ultrasound, angiography, surgical navigation equipment, tracking tools, ECG and associated software. Additionally and/or alternatively, the method of training the machine learning ML algorithm is for controlling image-guided therapy systems, as described herein. The method is implemented by a computer comprising a processor and a memory. Suitable computers are known. The method comprises obtaining training data including a set of image-guided therapy images (i.e. image data, particularly medical image data), respective gaze areas thereof and respective actions associated therewith from respective image-guided therapy procedures performed by respective clinicians on respective patients. Generally, a gaze area of an image is an area (also known as a point or a focus) of the image where a viewer is looking, as determined by measuring using eye tracking, for example. Hence, a gaze area of an image-guided therapy image comprises and/or is an intervention focus (also known as the focus area or the focus of the intervention) of the clinician (i.e. the viewer) and thus the area of that image upon which the clinician is focused. The method comprises training the ML algorithm using the training data comprising determining relationships between the image-guided therapy images, the respective gaze areas thereof and the respective actions associated therewith for the respective image-guided therapy procedures.

Advantageously, by learning the relationships between the image-guided therapy images, the respective gaze areas thereof and the respective actions associated therewith for the respective image-guided therapy procedures, the trained ML algorithm may infer focus areas of image-guided therapy images during image-guided therapy procedures performed by clinicians, rather than as determined by measuring using eye tracking, for example. Using the inferred focus areas, the trained ML algorithm may predict and enact actions associated with the inferred focus areas, thereby automating the assisting of image-guided therapy procedures and hence improving patient outcomes, without requiring use of eye tracking, for example.

In other words, during training, the ML algorithm learns gaze areas (and hence focus areas) of image-guided therapy images displayed by image-guided therapy systems and viewed by clinicians and actions associated therewith, while the clinicians are performing image-guided therapy procedures, including the actions associated therewith, and their respective gaze areas determined by measuring using eye tracking, for example. In contrast, during inference, the trained ML algorithm infers an inferred gaze area from an image-guided therapy image displayed by an image-guided therapy systems and viewed by a clinician, while the clinician is performing an image-guided therapy procedure but without determination of the respective gaze area by measuring using eye tracking, for example.

The method comprises obtaining training data including the set of image-guided therapy images, the respective gaze areas thereof and the respective actions associated therewith from respective image-guided therapy procedures performed by respective clinicians on respective patients.

The training data include the set of image-guided therapy images, the respective gaze areas thereof and the respective actions associated therewith. It should be understood that one or more image-guided therapy images are displayed to a clinician during an image-guided therapy procedure, for example on a display included in an image-guided therapy system. It should be understood that an image-guided therapy image may include a plurality of different scans, including scans acquired in real-time and scans acquired previously, together with patient and image-guided therapy system data. The particular focus (also known as intervention focus) of the clinician may depend on the step (also known as phase or stage) of the image-guided therapy procedure. For example, during a Percutaneous Coronary Intervention (PCI), also known as coronary stenting procedure or as angioplasty with stent, there may be several steps, such as: access though femoral, navigation of catheter up to the coronary tree ostium, assessment of the coronary tree (angiograms), wiring of one or several branches, navigation to stenosis and stenosis passing, stent positioning, stent delivery and many other examples of steps of the intervention within the intervention focus. Each of the intervention steps may be imaged and within each of the images, the gaze area and hence intervention focus may be determined by measuring using eye tracking, for example, for the training data. For example, during a wire navigation step of a PCI, the clinician will typically focus on a tip of the wire: During the wire navigation step, the clinician may enact an action, such as zoom a scan. Subsequently, during a treatment planning step, the clinician will typically focus on a lesion, to establish a correct stent size. In one example, the set of image-guided therapy images, the respective gaze areas thereof and the respective actions associated therewith include respective series (also known as a sequence) of image-guided therapy images and the respective gaze areas thereof from the respective image-guided therapy procedures. That is, one or more image-guided therapy images and respective gaze areas thereof may be obtained from a particular image-guided therapy procedure, for example a time series such as captured periodically (e.g. every 15, 30, 60 seconds), intermittently (e.g. upon manual capture at particular steps) or continuously (e.g. video). For example, image data of the currently displayed image-guided therapy image may be stored or a screenshot thereof captured.

Within the present context, the intervention focus (also known as the focus area or the focus of the intervention) is understood as a location (i.e. area, region) in an image at which a clinician would focus during (a step of) the intervention. In clinical practice, it is common to observe the clinician intensely focusing on a local region of an image i.e. the focus area.

In one example, the respective gaze areas are estimated using eye tracking of the respective clinicians. Eye tracking is known. Video-based eye trackers typically track, using a video camera, respective centers of the pupils of a viewer to estimate a gaze area of the viewer. Other eye-trackers are known. While such eye tracking is acceptable for obtaining training data, it is preferable to avoid eye tracking during routine image-guided therapy procedures due to additional cost, further complexity and/or the requirement for line of sight of the clinician. Hence, as described below, eye tracking is not required during inference.

In one example, obtaining the training data comprises displaying a first image-guided therapy image of the set thereof at a first time during a first image-guided therapy procedure performed by a first clinician and estimating a respective first gaze area of the first image-guided therapy image of the first clinician using eye tracking. For example, the first time may correspond with a particular step or be included in a series of image-guided therapy images, as described herein. In one example, obtaining the training data comprises displaying a second image-guided therapy image of the set thereof at a second time during the first image-guided therapy procedure performed by the first clinician and estimating a respective first gaze area of the second image-guided therapy image of the first clinician using eye tracking. In this way, a series of image-guided therapy images and the respective gaze areas thereof from a particular image-guided therapy procedure may be included in the training data.

The training data include respective actions, for example enacted by and/or on behalf of the respective clinicians, associated the image-guided therapy images and the respective gaze areas thereof from the respective image-guided therapy procedures. That is, in response to viewing an image-guided therapy image, particularly a gaze area thereof, a clinician may enact an associated action, typically relating to image processing and/or image-guided therapy system control, as described below. The action may be reactive, for example to address an observation, or proactive, for example in anticipation of a subsequent step. Conversely, in response to viewing an image-guided therapy image, particularly a gaze area thereof, a clinician may not enact an associated action i.e. a null action or absence of an action.

For example, during a wire navigation step of a PCI, the clinician will typically focus on a tip of the wire. During the wire navigation step, the clinician may enact an action, such as zoom the gaze area. Subsequently, during a treatment planning step, the clinician will typically focus on a lesion, to establish a correct stent size. During the treatment planning step, the clinician may enact an action, such as measure an extent of the lesion and/or a diameter of a vessel, for example using functionality provided via a mouse or a touchscreen tablet included in the image-guided therapy system.

In one example, the respective actions include image processing actions and/or image-guided therapy system control. Image processing actions may include zooming, panning, resizing, reordering, replacing, adding or removing a scan, image enhancement, image analysis including measurement. Image-guided therapy system control actions may include acquiring a scan, collimating an X-ray beam to adjust a dosage for imaging and controlling an orientation of an imaging device for acquiring a scan, and/or moving of a therapeutic device either manually or by a device driving mechanism. Other actions are known. It should be understood that these actions are typically enacted via the respective image-guided therapy systems and hence are stored with the image-guided therapy images and may be included with the training data. Additionally and/or alternatively, these actions may be logged manually and included with the training data.

In one example, the training data include speech and/or metadata associated with the image-guided therapy images, the respective gaze areas thereof and the respective actions associated therewith and optionally, respective image-guided therapy system data, from the respective image-guided therapy procedures. For example, a particular clinician may verbally comment during a particular image-guided therapy procedure, such as an observation in response to viewing a particular image-guided therapy image or a request to enact an action. The speech may be included in the training data manually or via automatic speech recognition, for example. For example, the metadata may include an imaging protocol, log information of executed algorithms, information on procedure time (e.g. time since the image-guided therapy procedure started), image-guided therapy system settings, image-guided therapy procedure type, name of the clinician, and/or patient data (e.g. from PACS).

In one example, the training data include auxiliary data, for example from auxiliary equipment such as ultrasound, angiography, surgical navigation equipment, tracking tools and associated software. Typically, such auxiliary equipment is communicatively coupled to the image-guided therapy system.

It should be understood that an image-guided therapy procedure is an instance (i.e. a particular intervention performed by a particular clinician on a particular patient) of a type of image-guided therapy, for example Percutaneous Coronary Intervention (PCI) such as for patients with coronary artery disease, peripheral artery disease or lead extraction indications; Cardiac Electrophysiology (EP); and Percutaneous Ablation (PA) (also known as image-guided ablation (IGA) or image-guided tumor-ablation). Other image-guided therapies are known. In one example, the respective image-guided therapy procedures are of the same image-guided therapy, for example for only PCI. In this way, the ML algorithm is trained specifically for a particular image-guided therapy. In one example, the respective image-guided therapy procedures are performed by the same clinician, for example an expert of a particular image-guided therapy. In this way, the ML algorithm is trained using training data obtained from the expert. In one example, the respective image-guided therapy procedures are performed according to a particular protocol. In this way, the ML algorithm is trained for that particular protocol, thereby improving standardization.

The method comprises training the ML algorithm using the training data comprising determining relationships between the image-guided therapy images, the respective gaze areas thereof and the respective actions associated therewith for the respective image-guided therapy procedures. That is, the ML algorithm learns that a particular gaze area of a clinician, and hence the focus area, is related to a particular image-guided therapy image, for example at a particular step and/or a particular time of the particular image-guided therapy procedure. In this way, the ML algorithm more generally learns the respective gaze areas, and hence respective focus areas, of respective image-guided therapy images, for example as a function of step and/or time of an image-guided therapy procedure. In other words, the ML algorithm learns about image-guided therapy procedures, including focus areas and optionally associated actions, from image-guided therapy images displayed to clinicians. Using this learning, the trained ML algorithm may be used to assist image-guided therapy procedure, as described herein.

In one example, determining relationships between the image-guided therapy images, the respective gaze areas thereof and the respective actions associated therewith for the respective image-guided therapy procedures comprises establishing respective intervention foci using the respective gaze areas, as described herein.

In one example, determining the relationships between the image-guided therapy images, the respective gaze areas thereof and the respective actions associated therewith for the respective image-guided therapy procedures comprises identifying features in the image-guided therapy images and relating the identified features to the respective gaze areas. Feature identification (also known as feature extraction) from images is known. That is, the ML algorithm learns to relate identified features to the respective gaze areas. In one example, the features include endogenous features (i.e. patient features for example physiological or anatomical features such as vessels, lesions, etc.) and exogenous features (i.e. image-guided therapy procedure features for example wires, leads, etc.)

In one example, training the ML algorithm using the training data comprises selectively weighting the training data. For example, some or all of the training data may be weighted based on a step of a respective image-guided therapy procedure, an enacted action, speech and/or metadata associated with the respective image-guided therapy image. In this way, an accuracy of training the ML algorithm may be improved. For example, if the speech indicates that the clinician has made an important observation or diagnosis, the corresponding gaze area (such as at the current time) and/or preceding gaze areas (such as at previous times, for example immediately before the current time) may be weighted relatively higher.

In one example, training the ML algorithm comprises pre-training the ML algorithm using training data obtained from a first type of image-guided therapy and subsequently, training the pre-trained algorithm using training data obtained from a second (different) type of image-guided therapy. In one example, training the ML algorithm comprises training the ML algorithm using training data obtained from a first type of image-guided therapy and subsequently, further training the trained algorithm using training data obtained from image-guided therapy procedures of the first type of image-guided therapy performed by a particular clinician or particular clinicians, for example at a particular hospital such as using a particular image-guided therapy system or a particular type of image-guided therapy system. In this way, the ML algorithm may be trained initially for the first type of image-guided therapy and subsequently, personalized or individualized for a particular protocol such as followed by particular clinicians at a particular hospital by further training.

### Suitable ML algorithms are known. In one example, the ML algorithm comprises and/or is a neural network (NN), a convolutional neural network (CNN) and/or a deep neural network (DNN)

According to a second aspect of the invention, there is provided a method of assisting an image-guided therapy procedure, for example as described with respect to the first aspect, mutatis mutandis. Additionally and/or alternatively, the method is of controlling an image-guided therapy system, as described herein. The method is implemented by a computer comprising a processor and a memory. Suitable computers are known. The method comprises acquiring an image-guided therapy image during the image-guided therapy procedure, for example from the image-guided therapy system and optionally, as displayed to a clinician performing the image-guided therapy procedure, generally as described with respect to the first aspect, mutatis mutandis. The method comprises inferring, using a trained machine learning (ML) algorithm, a first focus area of the image-guided therapy image. The trained ML algorithm may be trained according to the method of the first aspect. That is, the first focus area of the image-guided therapy image is inferred by the ML algorithm rather than determined by measurement using eye tracking, for example, as included with the training data described with respect to the first aspect. In other words, the first focus area is not inferred from eye tracker video camera data, for example, of the clinician but is instead inferred from the image-guided therapy image itself. That is, the inferred first focus area is what an expert, for example, would be focused on during the image-guided therapy procedure i.e. the intervention focus. In one example, the method does not comprise eye tracking i.e. the method excludes eye tracking. The method comprises assisting the image-guided therapy procedure based on the inferred first focus area. For example, the inferred first focus area may be highlighted in the image-guided therapy image, so as to draw attention of the clinician thereto and/or a predicted action may be enacted, for example as described with respect to the first aspect mutatis mutandis.

Advantageously, the trained ML algorithm infers focus areas (and hence intervention foci) of image-guided therapy images during image-guided therapy procedures performed by clinicians, rather than as determined by measuring using eye tracking, for example. Using the inferred focus areas, the trained ML algorithm may predict and enact actions associated with the inferred focus areas, thereby automating the assisting of image-guided therapy procedures and hence improving patient outcomes, without requiring use of eye tracking, for example. In this way, less experienced clinicians (and in turn their patients) may benefit from the experience and/or expertise of more experienced and/or more expert clinicians, for example to guide image analysis and/or interpretation. For example, an inferred gaze area may be highlighted to the clinician even though the clinician had not noted an importance thereof. Additionally and/or alternatively, clinicians (irrespective of experience) may benefit from automated enaction of actions, such as automatically measuring an extent of an identified lesion and/or a diameter of a vessel, thereby facilitating the establishment of a correct stent. Additionally and/or alternatively, the trained ML algorithm may be used to control a robot included in the image-guided therapy procedure, for example wherein the robot is configured to perform the image-guided therapy procedure, rather than a clinician or assisting a clinician. Hence, it should be understood that display of the image-guided therapy image is not necessarily required but instead, the inferred first focus area may be inferred from image data i.e. entirely in silico.

In one example, assisting the image-guided therapy procedure based on the inferred first focus area comprises displaying the image-guided therapy image including indicating, for example continuously indicating, the inferred first focus area. For example, the inferred first focus area may be highlighted in the displayed image-guided therapy image, such as by including a bounding box, so as to draw attention of the clinician thereto. By continuously indicating the inferred first focus area, the clinician is continuously assisted or guided during the image-guided therapy procedure.

In one example, assisting the image-guided therapy procedure based on the inferred first focus area comprises predicting, using the trained ML algorithm, an action associated the image-guided therapy image and the inferred first focus area and enacting the predicted action. In this way, the predicted action is enacted automatically, for example reactively or proactively, as described with respect to the first aspect, mutatis mutandis. Generally, the predicted actions may be as described with respect to the first aspect, mutatis mutandis. In one example, enacting the action comprises and/or is processing the image-guided therapy image, as described with respect to the first aspect. In one example, enacting the action comprises and/or is controlling the image-guided therapy procedure, as described with respect to the first aspect. In one example, enacting the action comprises conditionally enacting the action, for example conditionally upon authorization of the clinician. For example, if the action comprises and/or is controlling the image-guided therapy procedure, a graphical user interface may be displayed, prompting the clinician to authorize or cancel the action. In this way, unexpected re-orientation of the imaging device may be avoided or re-orientation of the imaging device may be warned or anticipated, for example.

In one example, the method comprises inferring, using the trained ML algorithm, a second focus area of the image-guided therapy image and assisting the image-guided therapy procedure based on the inferred second focus area. That is, a plurality of focus areas of the image-guided therapy image may be inferred. For example, during a wire navigation step of a PCI, the inferred first focus area may be a tip of the wire since the ML algorithm is trained that a clinician will typically focus on a tip of the wire. However, the image-guided therapy image may also include an important observation, as learned by the ML algorithm during training. Hence, the trained ML algorithm may indicate the second inferred gaze area and/or predict and enact an action based on the second inferred gaze area, is described with respect to the first inferred gaze area, mutatis mutandis. In one example, the method comprises prioritizing the inferred first focus area and the inferred second focus area, for example based on a risk to the patient and/or an importance to the image-guided therapy procedure.

According to a third aspect of the invention, there is provided an image-guided therapy system, including a computer comprising a processor and a memory, and an eye tracker, configured to implement a method according to the first aspect.

According to a fourth aspect of the invention, there is provided an image-guided therapy system, including a computer comprising a processor and a memory, configured to implement a method according to the second aspect.

According to a fifth aspect of the invention, there is provided a computer comprising a processor and a memory configured to implement a method according to the first aspect and/or the second aspect, a computer program comprising instructions which, when executed by a computer comprising a processor and a memory, cause the computer to implement a method according to the first aspect and/or the second aspect, a non-transient computer-readable storage medium comprising instructions which, when executed by a computer comprising a processor and a memory, cause the computer to implement a method according to the first aspect and/or the second aspect or a machine learning, ML, algorithm trained according to the method according to the first aspect.

All disclosures as described here in relation to any aspect of the invention applies equally to all other aspects of the invention.

In the following, examples and embodiments of the invention are described with reference to the figures.

### BRIEF DESCRIPTION OF THE DRAWINGS

Fig. 1 schematically depicts a method of training a machine learning algorithm for assisting image-guided therapy procedures, according to an exemplary embodiment;
Fig. 2 schematically depicts a method of assisting an image-guided therapy procedure, according to an exemplary embodiment;
Fig. 3 schematically depicts a method of training a machine learning algorithm for assisting image-guided therapy procedures, according to an exemplary embodiment; and
Fig. 4 schematically depicts a method of assisting an image-guided therapy procedure, according to an exemplary embodiment.

### DETAILED DESCRIPTION OF EMBODIMENTS

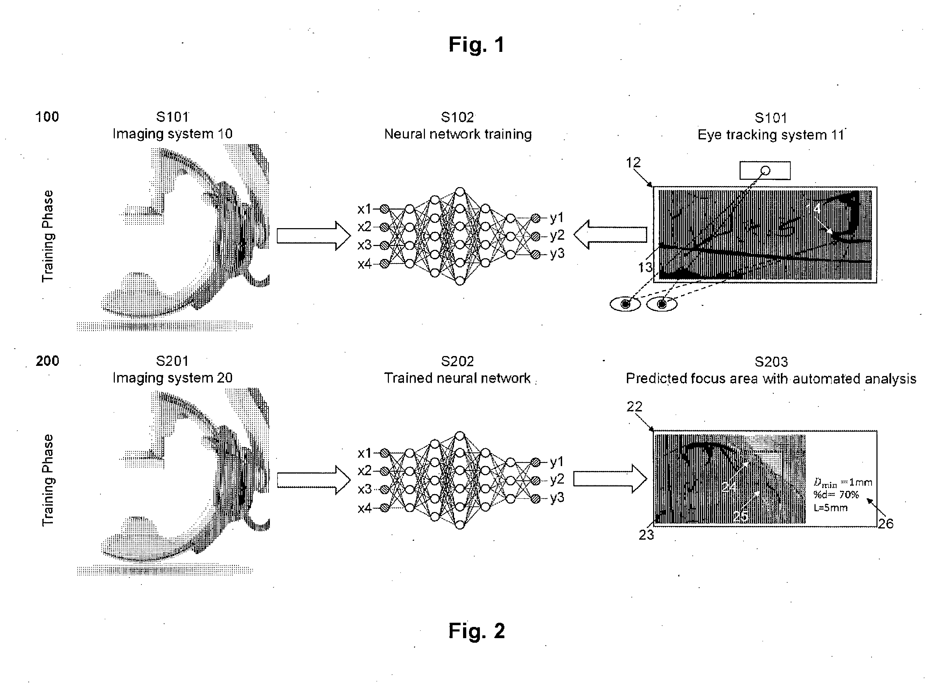

Fig. 1 schematically depicts a method 100 of training a machine learning algorithm for assisting image-guided therapy procedures, according to an exemplary embodiment. The method 100 is implemented by a computer comprising a processor and a memory. The method 100 comprises step S101 of obtaining training data including a set of image-guided therapy images and respective gaze areas thereof from respective image-guided therapy procedures performed by respective clinicians on respective patients. The method 100 comprises step S102 of training the machine learning algorithm using the training data comprising determining relationships between the image-guided therapy images, the respective gaze areas thereof and the respective actions associated therewith for the respective image-guided therapy procedures. The method 100 may include any step described with respect to the first aspect.

Advantageously, by learning the relationships between the image-guided therapy images, the respective gaze areas thereof and the respective actions associated therewith for the respective image-guided therapy procedures, the trained ML algorithm may infer focus areas of image-guided therapy images during image-guided therapy procedures performed by clinicians, rather than as determined by measuring using eye tracking, for example. Using the inferred focus areas, the trained ML algorithm may predict and enact actions associated with the inferred focus areas, thereby automating the assisting of image-guided therapy procedures and hence improving patient outcomes, without requiring use of eye tracking, for example.

In this example, the image-guided therapy 10 is a Philips (RTM) Azurion (RTM) image-guided therapy system (available from Koninklijke Philips N.V., NL). In this example, the respective gaze areas are estimated using eye tracking of the respective clinicians, using an eye tracker 11, particularly an EyeWorks (RTM) + FX3 eye tracker (available from EyeTracking, Inc., CA, USA). In this example, the method comprises obtaining the training data comprises displaying a first image-guided therapy image 13 of the set thereof on a display 12 at a first time during a first image-guided therapy procedure performed by a first clinician and estimating a respective first gaze area 14 of the first image-guided therapy image of the first clinician using eye tracking.

In this example, the training data include respective actions, for example enacted by and/or on behalf of the respective clinicians, associated the image-guided therapy images, the respective gaze areas thereof and the respective actions associated therewith from the respective image-guided therapy procedures. For example, during a wire navigation step of a PCI, the clinician will typically focus on a tip of the wire. During the wire navigation step, the clinician may enact an action, such as zoom the gaze area. Subsequently, during a treatment planning step, the clinician will typically focus on a lesion, to establish a correct stent size. During the treatment planning step, the clinician may enact an action, such as measure an extent of the lesion and/or a diameter of a vessel, for example using functionality provided via a mouse or a touchscreen tablet included in the image-guided therapy system. In this example, the respective actions include image processing actions and/or image-guided therapy system control. Image processing actions may include zooming, panning, resizing, reordering, replacing, adding or removing a scan, image enhancement, image analysis including measurement. Image-guided therapy system control actions may include acquiring a scan, collimating an X-ray beam to adjust a dosage for imaging and controlling an orientation of an imaging device for acquiring a scan.

In this example, the respective image-guided therapy procedures are of the same image-guided therapy, particularly only PCI. In this example, the respective image-guided therapy procedures are performed by the same clinician, for example an expert of a particular image-guided therapy.

In this example, determining relationships between the image-guided therapy images, the respective gaze areas thereof and the respective actions associated therewith for the respective image-guided therapy procedures comprises establishing respective focus areas using the respective gaze areas, as described herein.

In this example, determining the relationships between the image-guided therapy images, the respective gaze areas thereof and the respective actions associated therewith for the respective image-guided therapy procedures comprises identifying features in the image-guided therapy images and relating the identified features to the respective gaze areas. That is, the ML algorithm learns to relate identified features to the respective gaze areas. In this example, the features include endogenous features (i.e. patient features for example physiological or anatomical features such as vessels, lesions, etc.) and exogenous features (i.e. image-guided therapy procedure features for example wires, leads, etc.)

In this example, the ML algorithm is a neural network.

Fig. 2 schematically depicts a method 200 of assisting an image-guided therapy procedure, according to an exemplary embodiment. The method 200 is implemented by a computer comprising a processor and a memory. The method 200 comprises step S201 of acquiring an image-guided therapy image during the image-guided therapy procedure. The method 200 comprises step S202 of inferring, using a trained machine learning algorithm, a first focus area of the image-guided therapy image. The method 200 comprises step S203 of assisting the image-guided therapy procedure based on the inferred first focus area. The method 200 may include any step described with respect to the second aspect.

Advantageously, the trained ML algorithm infers focus areas of image-guided therapy images during image-guided therapy procedures performed by clinicians, rather than as determined by measuring using eye tracking, for example. Using the inferred focus areas, the trained ML algorithm may predict and enact actions associated with the inferred focus areas, thereby automating the assisting of image-guided therapy procedures and hence improving patient outcomes, without requiring use of eye tracking, for example.

That is, the first focus area of the image-guided therapy image is inferred by the ML algorithm rather than determined by measurement using eye tracking, for example, as included with the training data described with respect to the first aspect. In other words, the first focus area is not inferred from eye tracker video camera data, for example, of the clinician but is instead inferred from the image-guided therapy image itself. That is, the inferred first focus area is what an expert, for example, would be focused on during the image-guided therapy procedure i.e. the intervention focus. In this example, the method 200 does not comprise eye tracking i.e. the method 200 excludes eye tracking.

In this example, the trained ML algorithm is trained according to the method 100 described with respect to Fig. 1.

In this example, the image-guided therapy 20 is a Philips (RTM) Azurion (RTM) image-guided therapy system (available from Koninklijke Philips N.V., NL).

In this example, assisting the image-guided therapy procedure based on the inferred first focus area comprises displaying the image-guided therapy image 23 on a display 22 including indicating, for example continuously indicating, the inferred first focus area. In this example, the inferred first focus area is highlighted in the displayed image-guided therapy image, by including a bounding box 25, so as to draw attention of the clinician thereto. By continuously indicating the inferred first focus area, the clinician is continuously assisted or guided during the image-guided therapy procedure.

In this example, assisting the image-guided therapy procedure based on the inferred first focus area comprises predicting, using the trained ML algorithm, an action associated the image-guided therapy image and the inferred first focus area and enacting the predicted action. In this example, the action comprises measuring an extent of a lesion and a diameter of a vessel 24 and selecting a correct stent 26.

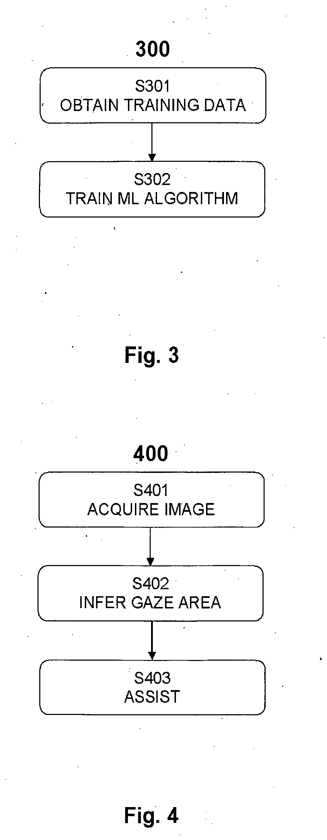

Fig. 3 schematically depicts a method 300 of training a machine learning algorithm for assisting image-guided therapy procedures, according to an exemplary embodiment. The method 300 is implemented by a computer comprising a processor and a memory. The method 300 comprises step S301 of obtaining training data including a set of image-guided therapy images, respective gaze areas thereof and respective actions associated therewith from respective image-guided therapy procedures performed by respective clinicians on respective patients. The method 300 comprises step S302 of training the machine learning algorithm using the training data comprising determining relationships between the image-guided therapy images, the respective gaze areas thereof and the respective actions associated therewith for the respective image-guided therapy procedures. The method 300 may include any step described with respect to the first aspect.

Fig. 4 schematically depicts a method 400 of assisting an image-guided therapy procedure, according to an exemplary embodiment. The method 400 is implemented by a computer comprising a processor and a memory. The method 400 comprises step S401 of acquiring an image-guided therapy image during the image-guided therapy procedure. The method 400 comprises step S402 of inferring, using a trained machine learning algorithm, a first focus area of the image-guided therapy image. The method 400 comprises step S403 of assisting the image-guided therapy procedure based on the inferred first focus area. The method 400 may include any step described with respect to the second aspect.

Where an indefinite or definite article is used when referring to a singular noun, e.g. "a", "an" or "the", this includes the plurality of the noun unless something else is specifically stated. It is to be understood that the terms so used are interchangeable under appropriate circumstances and that the embodiments of the invention described here are capable of operating in other sequences than described or illustrated herein.

## Claims

1. A method of training a machine learning algorithm for assisting image-guided therapy procedures, the method implemented by a computer comprising a processor and a memory, the method comprising:
obtaining training data including a set of image-guided therapy images, respective gaze areas thereof and respective actions associated therewith from respective image-guided therapy procedures performed by respective clinicians on respective patients (S101, S301); and
training the machine learning algorithm using the training data comprising determining relationships between the image-guided therapy images, the respective gaze areas thereof and the respective actions associated therewith for the respective image-guided therapy procedures (S102, S302).

2. The method according to claim 1, wherein obtaining the training data comprises displaying a first image-guided therapy image of the set thereof at a first time during a first image-guided therapy procedure performed by a first clinician and estimating a respective first gaze area of the first image-guided therapy image of the first clinician using eye tracking.

3. The method according to any previous claim, wherein the respective actions include image processing.

4. The method according to claim 3, wherein the respective actions include image-guided therapy system control.

5. The method according to any previous claim, wherein the training data include speech and/or metadata associated with the image-guided therapy images, the respective gaze areas thereof and the respective actions associated therewith from the respective image-guided therapy procedures.

6. The method according to any previous claim, wherein determining the relationships between the image-guided therapy images, the respective gaze areas thereof and the respective actions associated therewith for the respective image-guided therapy procedures comprises identifying features in the image-guided therapy images and relating the identified features to the respective gaze areas.

7. The method according to any previous claim, wherein training the ML algorithm using the training data comprises selectively weighting the training data.

8. A method of assisting an image-guided therapy procedure, the method implemented by a computer comprising a processor and a memory, the method comprising:
acquiring an image-guided therapy image during the image-guided therapy procedure (S201; S401);
inferring, using a trained machine learning algorithm, a first focus area of the image-guided therapy image (S202, S402); and
assisting the image-guided therapy procedure based on the inferred focus gaze area (S203, S403).

9. The method according to claim 8, wherein assisting the image-guided therapy procedure based on the inferred first focus area comprises:
displaying the image-guided therapy image including indicating the inferred first focus area.

10. The method according to any of claims 8 to 9, wherein assisting the image-guided therapy procedure based on the inferred first focus area comprises:
predicting, using the trained machine learning algorithm, an action associated the image-guided therapy image and the inferred first focus area; and
enacting the predicted action.

11. The method according to claim 10, wherein enacting the action comprises and/or is processing the image-guided therapy image.

12. The method according to any of claims 8 to 11, wherein enacting the action comprises and/or is controlling the image-guided therapy procedure.

13. The method according to any of claims 8 to 12, comprising:
inferring, using the trained machine learning algorithm, a second focus area of the image-guided therapy image; and
assisting the image-guided therapy procedure based on the inferred second focus area.

14. An image-guided therapy system, including a computer comprising a processor and a memory, configured to implement a method according to any of claims 8 to 13.

15. A computer comprising a processor and a memory configured to implement a method according to any of claims 1 to 13, a computer program comprising instructions which, when executed by a computer comprising a processor and a memory, cause the computer to implement a method according to any of claims 1 to 13, a non-transient computer-readable storage medium comprising instructions which, when executed by a computer comprising a processor and a memory, cause the computer to implement a method according to any of claims 1 to 13 or a machine learning, ML, algorithm trained according to the method according to any of claims 1 to 7.
